# EUROPEAN PATENT SPECIFICATION

(11) **EP 0 921 817 B1**
(45) Date of publication and mention of the grant of the patent: **28.03.2001**
(21) Application number: 98901405.5
(22) Date of filing: 28.01.1998
(51) Int. Cl.: A61K 47/48

(54) **PEGYLATION PROCESS**
PEGYLATIONSVERFAHREN
PROCEDE DE P.E.G.YLATION

(30) Priority: 29.01.1997 GB 9701800; 29.01.1997 GB 9701804; 06.03.1997 GB 9704653; 22.04.1997 GB 9708055
(43) Date of publication of application: 16.06.1999
(73) Proprietor: PolyMASC Pharmaceuticals plc, London NW3 2EZ (GB)
(72) Inventor: FRANCIS, Gillian, Elizabeth, Berkshire RG4 9GH (GB); FISHER, Derek, Hertfordshire AL3 4HY (GB); MALIK, Farooq, London SW16 2TS (GB)
(74) Representative: Nachshen, Neil Jacob
(86) International application number: GB9800253
(87) International publication number: WO9832466

(56) References cited:
- EP-A- 0 539 167
- WO-A-90/04650
- WO-A-95/06058
- WO-A-95/34326
- FRANCIS G E ET AL: "POLYETHYLENE GLYCOL MODIFICATION: RELEVANCE OF IMPROVED METHODOLOGY TO TUMOUR TARGETING" JOURNAL OF DRUG TARGETING, vol. 3, 1996, pages 321-340, XP002058312
- DELGADO C. ET AL: "Polymer derivatized proteins: analytical and preparative problems" PHARMACEUTICAL SCIENCES, vol. 3, 1997, pages 59-66, XP002068522
- ZALIPSKY S: "CHEMISTRY OF POLYETHYLENE GLYCOL CONJUGATES WITH BIOLOGICALLY ACTIVE MOLECULES" ADVANCED DRUG DELIVERY REVIEWS, vol. 16, no. 2/03, 1995, pages 157-182, XP002037428
- ZALIPSKY S.: "Functionalized poly(ethylene glycol) for preparation of biologically relevant conjugates" BIOCONJUGATE CHEM., vol. 6, no. 2, 1995, pages 150-165, XP002068523 cited in the application
- VERONESE F. M. ET AL: "A comparative study of enzymatic, structural and pharmacokinetic properties of superoxide dismutase isolated from two sources and modified by monomethoxypolyethylene glycol using different methods of coupling" ANNALS N. YORK ACAD. SCI., vol. 613, 1990, NEW YORK, pages 468-474, XP002068524
- GAERTNER H F ET AL: "SITE-SPECIFIC ATTACHMENT OF FUNCTIONALIZED POLY(ETHYLENE GLYCOL) TOTHE AMINO TERMINUS OF PROTEINS" BIOCONJUGATE CHEMISTRY, vol. 7, no. 1, 1996, pages 38-44, XP000646874

## Description

The present invention relates to the attachment of a polyethylene glycol (PEG) moiety to a target substrate. Processes for such attachment will be hereinafter referred to as "PEGylation" of the substrate. In particular, the present invention relates to a process for direct covalent PEGylation of a substrate. comprising the reaction of a halogenated PEG with the substrate wherein the halogen of the halogenated PEG acts as a leaving group in the PEGylation reaction.

Covalent attachment of PEG to molecules such as proteins or structures such as liposomes is well known to improve their pharmacological and physiological properties.

EP-A-354855 describes a liposome which comprises a PEG-bound phospholipid wherein the PEG moiety is bonded to a phospholipid present in the liposome membrane. This is claimed to provide a reduction in the absorption of proteins to the liposome *in vivo* and hence an increase in its *in vivo* stability.

EP-A-154316 describes a method for chemically modifying lymphokines by attachment of a PEG moiety wherein the PEG is bonded to at least one primary amino group of the lymphokine. This is claimed to result in the delayed clearance of lymphokines when used as drugs and to decrease their antigenicity.

There are many methods for achieving covalent coupling of PEG to substrates. All such methods require the activation of the PEG by attachment of a group usually referred to as an "activating moiety" or by converting a terminal moiety of the PEG into an activating moiety. This is followed by a second step where the PEG couples to the target molecule, usually via a residual portion of the activating moiety which may be referred to as the "coupling moiety".

Examples of known techniques include:
**Succinimidyl Active Ester Methods:** see e.g. US Patent 4,412,989; WO 86/04145; WO 87/00056; EP-A-0 247 860, C. Monfardini, O. Shiavon, P. Caliceti, M. Morpurgo, J. M. Harris, and F. M. Veronese, "A branched monomethoxypoly(ethylene glycol) for protein modification," Bioconjugate Chem., 6,62-69 (1995), Zalipsky, S. et al. (1991) in "Polymeric Drugs and Drug Delivery Systems" (R. L. Dunn & R. M. Ottenbrite, eds.) ACS, Washington, DC, Chapter 10, Zalipsky, S. et al. (1992) Biotechnol. Appl. Biochem. **15**:100, Chiu, H.-C. et al. (1993) Bioconjugate Chem. **4**:290, Sirokman, G. & Fasman, G. (1993) Protein Sci. **2**:1161, Veronese, F. M. et al (1989) J. Controlled Release **10**:145, Abuchowski, A. et al (1984) Cancer Biochem. Biophys. **7**:175, Joppich, M. & Luisi, P.L. (1979) Macromol. Chem. **180**:1381, Klibanov, A. L. et al (1990) FEBS Letters **268**:235, Sartore, L. et al (1991) Appl. Biochem. Biotech. 31:213
**Carbonyldiimidazole Method:** see e.g. EP-A-0 154 432.
**Phenylchloroformate Methods:** see e.g. WO 89/06546 and WO 90/15628.
**PEG-Succinate Mixed Anhydride Methods:** see e.g. Ahlstedt et al (1983) Int. Arch. Allergy Appl. Immunol., 71,228-232; Richter and Akerblom (1983) Int. Arch. Allergy Appl. Immunol, 70, 124-131;
**Organic Sulphonyl Halide Methods:** see e.g. US Patent 4,415,665.
**PEG-Maleimide and Related Methods:** see e.g. Goodson & Katre (1990) Biotechnology, 8, 343-346.
**Phenylglyoxal Method:** see e.g. EP-A-0 340 741
**Succinimide Carbonate Method:** see e.g. WO 90/13540; WO 91/07190
**Cyanogen Bromide Method:** see USP 4,301,144
**Poly-PEG Maleic Acid Anhydride Method:** Yoshimoto et al (1987) Biochem. and Biophy. Res. Commun. 148, 876-882.
**Cyanuric chloride method:** Abuchowski, A. van Es, T., Palczuk, N.C., & David. F.F. (1977). Alteration of immunological properties of bovine serum albumin by covalent attachment of polyethylene glycol. J. Biol. Chem., 252, 3578-3581.
**PEG acetaldehyde methods:** Royer, G.P. US 4,002,531 EP-A-0154316. Harris, J. M., Yoshinaga. K. Paley, M.S., & Herati, M. R. (1989). New activated PEG derivatives for affinity partitioning. In D. Fisher & I.A. Sutherland (Eds) Separations Using Aqueous Phase Systems. Applications in Cell Biology and Biotechnology (pp. 203-210). London Plenum Press.
**Amine acylation methods (both PEG-COOH and PEG-NH**_{**2**}**)**: see e.g. EP0072111 and EP 0401384.
**Vinylsulfone method:** M. Morpurgo, F. M. Veronese, D. Kachensky and J. M. Harris, J. Bioconj. Chem., 7, 363-368 (1996).
**PEG epoxide methods:** Elling, L. & Kula, M-R. (1991) Biotech. Appl. Biochem. **13**.354.
**PEG isocyanate method:** R. B. Greenwald. A. Pendri and D. Bolikal, J. Org. Chem., **60**, 331-336 (1995).
**PEG orthopyridyl-disulphide:** C. Woghiren, B. Sharma and S. Stein, Bioconj. Chem., 4,314 (1993).
**PEG-proprionaldehyde:** Harris, J.M., Dust, J.M., McGill, Harris, P.A., Edgell, M.J., Sedaghat-Herati, R.M., Karr, L.J., & Donnelly, D.L. (1991). New polyethylene glycols for biomedical applications. Chapter 27 in S.W. Shalaby, C. L. McCormick. & G. B. Butler (Eds.), Water-Soluble Polymers Washington D.C.: American Chemical Society.

These methods suffer from one or more of the following defects: Substantial loss of biological activity (e.g. 20-95% loss of bio-activity) is frequently seen with the cyanuric chloride method:
Savoca KV, Abuchowski A, van Es T, Davis FF, Palczuk NC (1979), Biochem Biophys Acta 578: 47-53, Ashihara Y, Kono T, Yamazaki S, Inada Y (1978) Biochem Biophys Res Commun 83:385-391, Kamisaki Y, Wada H, Yagura T, Matsushima A, Inada Y (1981) J Pharmacol Exp Ther 216: 410-414, Wieder K. J. Palczuk NC, van Es T, Davis F F (1979) J Biol Chem 254:12579-12587, Nishimura H, Matsushima A, Inada Y (1981) Enzyme 26:49-53 and Pyatak PS, Abuchowski A, Davis FF (1980) Res Commun Chem Pathol Pharmacol 29:113-127
The coupling of PEG (or other polymers) to proteins (or other target molecules) is, with few exceptions, in a manner which leaves part of the activating moiety, a coupling moiety, between the PEG and the target molecule. Of the above methods, only the organic sulphonyl halide methods and PEG-acetaldehyde methods disclosed in Royer US 4002531 (1977) and Harris (1989, ibid) couple PEG directly without coupling moieties i.e. to produce a "linkerless" PEGylated product. With the exception of some other PEG acetaldehyde methods where the coupling moiety is ethylene oxide (and thus indistinguishable from PEG itself) and the direct coupling methods above, all other coupling methods incorporate a coupling moiety distinct from the polymer and the target and are thus regarded as "indirect" coupling methods.

The incorporation of a coupling moiety generates further problems depending on the nature of the coupling moiety, thus
(i) some coupling moieties provide targets for enzymatic cleavage or hydrolysis (see below);
(ii) some coupling moieties provide an immunogenic/antigenic group (e.g. the triazine ring of the cyanuric chloride method or the succinyl group of the succinimidyl succinate method and PEG succinate mixed anhydride method);
(iii) some coupling moieties are potentially toxic or are themselves of unknown toxicity but derived from a compound known to be toxic (e.g. the triazine ring of the cyanuric chloride method and reagents in the phenylchloroformate method); and
(iv) some coupling moieties provide reactive groups capable of linking further molecules to the PEG-target construct via the coupling moiety (e.g. the triazine ring of the cyanuric chloride method, Leonard, M. et al., Tetrahedron, 40: 1585 (1984)) and,
(v) Some coupling groups alter surface charge at the site of attachment of the polymer.

Coupling in some instances is thus via an unstable bond liable to be cleaved by enzymes present in serum, plasma, cells or other biological materials or by procedures applied to the PEG-target product. This has two possible deleterious consequences,
(i) the PEG-target construct is degraded enzymatically or by the conditions required for subsequent reaction steps; the former occurs particularly with methods generating ester bonds and probably also with amide bonds; and
(ii) removal of the PEG moiety alters the target molecule; this occurs with some succinimidyl active ester and mixed anhydride methods,
and either or both of these can occur.

Many of the above methods recommend long coupling times and/or a non physiological pH for the PEGylation reaction, thus rendering some target molecules less active or inactive (cf. the cyanuric chloride, phenylchloroformate, acetaldehyde and propionaldehyde methods).

Many of these methods use activated PEG species and/or produce coproducts which are toxic in a wide range of bioassays and which are potentially toxic in vivo if not separated from the product (e.g. the phenylchloroformate, cyanuric chloride methods).

Some methods are unsuitable for use in aqueous solution, thus limiting the target molecules to those which will tolerate non-aqueous conditions (cf. the organic sulphonyl halide method using trifluoromethanesulphonyl chloride).

Some of the activated PEG-target constructs are unstable, for instance being subject to hydrolysis during either the activation or coupling reactions (cf. the phenylchloroformate method). For example, PEG acetaldehyde is sensitive to decomposition under basic conditions and can give inconsistent results.

Ouchi T., et al [(1987) J. Macromol. Sci. Chem. A24 1011-1032] discusses the PEGylation of 5-fluorouracil with various methoxy-PEG derivatives to generate methoxy-PEG ether, ester or amide-linked constructs. The preparation of methoxy-PEG-ether-5-fluorouracil from a methoxy-PEG-Br derivative in chlorobenzene using tetra-n-butylammonium bromide as a phase-transfer catalyst is described. None of the methoxy-PEG-ether-5-fluorouracil derivatives thus produced showed bioactivity (i.e. anti-tumour activity).

Zheng Hu et al (1987) Acta Pharmaceutical Sinica, 22 (8) 637 - 640 discusses the synthesis of PEG-estrogen compounds from chlorinated polyethylene in non-aqueous solvents using the Williamson reaction.

Probably the most advantageous PEGylation method employed hitherto is the TMPEG method, mentioned in WOA-90/04606, which comprises activation of monomethoxy PEG ("MPEG") with 2,2,2-trifluoroethanesulphonyl chloride (tresyl chloride) to produce tresyl MPEG ("TMPEG") which is subsequently reacted with a target protein molecule to produce monomethoxy PEGylated products. At physiological pH the TMPEG method is a "direct" coupling method in that the PEG moiety is coupled directly to the target substrate without a coupling or linker moiety. A similar technique is described in WO 90/04650 for coupling monomethoxy PEG moieties to DNA/protein complexes.

It is also known that the use of TMPEG as the activated PEG for use in PEGylation can, particularly at very high pH, result in the elimination of HF by an alternate pathway. This alternative elimination pathway may occur for example when reacting TMPEG with a protein and involves the elimination of HF which converts TMPEG into an intermediate alkene followed by hydration and further elimination of HF to create the acyl fluoride which is converted to the α-sulphonate acid via further hydrolysis. The alkene and acyl fluoride MPEG derivatives can react with target molecules to form a sulphonate amide derivative.

It is clearly desirable to develop a functionalised PEG which is simple and cheap to prepare, which can be used to PEGylate a wide range of potential substrates, which generates a linkerless or directly coupled PEGylated substrate, is capable of pegylating a substrate in both aqueous and non-aqueous solvents and which does not result in any of the undesirable side effects listed above. It is also desirable to have a PEGylation process which functions rapidly under physiological conditions since this is critical for retention of biological activity in the PEGylation of many proteins.

Hence there is provided according to the present invention a process *for* the PEGylation of a substrate comprising the reaction of a halogenated PEG of general formula I or general fomula II

X-PEG-Y (I)

(PEG)ₙ-Yᵢ (II)

wherein:
in formula I PEG is a bivalent group of formula -(-CH₂CH₂O-)ₘ-CH₂CH₂-, where m is equal or than 1, derived from a polyethylene glycol;
X is a halogen atom, a blocking group or an activating group capable of coupling the PEG moiety to another moiety; Y is a halogen; n represents the number of PEG termini and n is equal or greater than 2; i is equal or less than n; and i/n PEG temimi are substituted by Y in compounds of formula II, with the substrate wherein the halogen of the halogenated PEG acts as a leaving group and the PEG binds directly to the substrate, with the proviso that the substrate is not a steroid or when the halogenated PEG_{,} is PEG-bromide, the substrate is not 5-fluorouracil.

Halogenated PEGs of Formula I may be monofunctional, homobifunctional, or heterobifunctional activated PEGs i.e. a halogenated PEG of formula I may have two terminal halogens (X and Y are halogens which, may be the same or different), or when only one terminal halogen is present the other terminal group X may be either a blocking group or an activating group. Halogenated PEGs of Formula II may be of branched, cruciform or stellate structure. In preferred embodiments of the present invention, X is a blocking group selected from methyl, t-butyl and benzyl ethers.

In further preferred embodiments of the present invention, X is an activating group having an atom that is susceptible to nucleophilic attack or is capable of rendering the terminal carbon atom of the PEG susceptible to nucleophilic attack or equivalent alternative substitution and is preferably a sulphonate ester, a substituted triazine, a N-hydroxysuccinimide active ester, an anhydride, a substituted phenyl carbonate, oxycarbonylimidazole, a maleimide, an aldehyde, a glyoxal, carboxylate, a vinyl sulphone, an epoxide, an isocyanate, a disulphide, an acrylate, an allyl ether, a silane or a cyanate ester. More preferably X is an activating group selected from
2,2,2-trifluoroethanesulphonate,
pentafluorobenzenesulphonate,
fluorosulphonate,
2,4,5-trifluorobenzenesulphonate,
2,4-difluorobenzenesulphonate,
2-chloro-4-fluorobenzenesulphonate,
3-chloro-4-fluorobenzenesulphonate,
4-amino-3-chlorobenzenesulphonate,
4-amino-3-fluorobenzenesulphonate,
o-trifluoromethylbenzenesuplphonate,
m-trifluoromethylbenzenesulphonate,
p-trifluoromethylbenzenesulphonate,
2-trifluoromethoxybenzenesulphonate,
4-trifluoromethoxybenzenesulphonate,
5-fluoro-2-methylbenzenesulphonate,
4,6-dichlorotriazine,
6-chlorotriazine,
N-hydroxysuccinimidyl succinate,
N-hydroxysuccinimidyl glutarate,
N-hydroxysuccinimidyl succinamide,
N-hydroxysuccinimidylalkanedioicamides,
N-hydroxysuccinimidyl derivatives of carboxymethylated polymers,
succinimidylcarbonate,
N-hydroxysuccinimidyl esters of amino acids,
succinate mixed anhydride,
succinic anydride,
trichlorophenyl carbonate,
nitrophenyl carbonate,
maleimide,
N-substituted maleimide,
acetaldehyde,
propionaldehyde and chemically equivalent sulphur analogues,
glyoxal,
phenylglyoxal,
acrylate,
methacrylate.

Preferred halogens for the groups X and Y include chlorine, bromine and iodine. Chlorine is most preferred.

In the most preferred embodiments of the present invention, the halogenated PEG is one of
- monomethoxy PEG-Cl
- monomethoxy PEG-Br
- monomethoxy PEG-I
- Cl-PEG-Cl
- Br-PEG-Br
- I-PEG-I

Although some halogenated PEGs are known compounds, it is particularly surprising that they have utility as PEG derivatives suitable for direct use in a PEGylation reaction. It was previously believed that halogens would be of vastly inferior reactivity to known leaving groups such as tosylate or tresylate. For example McMurry J. in "Organic Chemistry" 4th Ed. (1996) cites chlorine as having 300 times less reactivity than tosylate as a leaving group. When considering that tresylate is described as having 100 fold greater reactivity than tosylate (March J. Advanced Organic Chemistry Reactions, Mechanisms and Structure, 4th Ed. [1992]), it may be concluded that chlorine would be expected to have 30,000 fold less reactivity than tresylate.

Halogenated PEGs may be synthesised by methods well known in the art. PEG may be synthesised or purchased commercially and then derivatised with halogen, activating groups or blocking groups, as required, using methods disclosed in e.g. Bayer E. et al, Polymer Bulletin 8,585 - 592 (1982); Zalipsky, S et al, Eur. Polym. J. Vol 19 No. 12 pp 1177-1183 (1983); Buckmann A. F., Morr. M and Johansson G., Makromol. Chem. 182, 1379-1384 (1981); Harris, J.M. J. Macromol. Sci., Rev. Polym. Chem. Phys. C25(3) 325-373 (1985); Harris, J.M., Struck, E. C., Case, M. G., et al. J. Poly. Sci, Poly. Chem. Ed. 22, 341-352 (1984); Zalipsky, S. & Lee, C. in Poly(Ethylene Glycol) Chemistry: Biotechnical and Biomedical applications (ed Harris, J. M.) 347-370 (Plenum Press, New York, 1992); and as reviewed in Zalipsky S. Bioconjugate Chem. (1995) 6 150-165 where MPEG-Cl was used to prepare activated PEGs which were subsequently linked to substrates.

Multi-halogenated PEGs can be constructed using either naturally branched PEGs, such as the cruciform PEG found in some preparations of high molecular weight PEGs, or from proprietary multibranched PEGs known as "star" PEGs. Derivatisation of the free PEG termini with halogen is achieved as for halogenated PEGs above.

Reaction conditions for the process of the present invention will clearly depend upon the nature of X, Y and of the substrate.

As indicated above, the PEG moieties of the halogenated PEG's used in accordance with the invention, may desirably be derived from commercially available PEGs. These materials are generally characterised by their number and weight average molecular weight. For example, PEG-5000 is a polyethylene glycol having a number average molecular weight of about 5000. The size of the PEG moiety to be attached to the target substrate will usually be chosen according to the nature of the substrate and how its properties are desired to be modified by the attachment of the PEG moiety. For example, if the target substrate is a liposome for administration to an animal and it is desired to increase the circulation half life of the liposome after administration, a PEG of molecular weight 1000 to 5000 may be selected. It should be noted, however, that the process of the present invention is generally applicable to the attachment of PEG moieties of any size to target substrates.

PEGylated substrates generated according to the present invention particularly include those which do not lose their bioactivity relative to the unPEGylated substrate. Thus PEGylation according to the present invention may maintain or increase the specific activity of a substrate or it may increase the *in vivo* half-life of a substrate which has had its specific activity decreased, maintained or increased by PEGylation. Additionally PEGylation according to the present invention may differentially modify the specific activity of pleiotropic substrates such as certain proteins.

The term "substrate" as used herein is intended to include any molecule, macromolecule or structure which is capable of being covalently attached to a PEG moiety and which thereby may have its chemical, biological, physiological or physical properties modified. It is not intended to encompass molecules which when reacted with halogenated PEG merely produce a further activated PEG derivative which is to be used as an intermediate to couple the PEG moiety to another substrate. The substrate is not a steroid.

Suitable substrates to which PEG can be attached in accordance with the present invention include materials having biological activity which are useful in, for instance diagnosis or therapy and which are all well known to those skilled in the art. They all contain at least one group capable of reacting with the halogenated PEG. Examples of such reactive groups include primary, secondary and tertiary amino groups, thiol groups and aromatic hydroxy groups.

More specifically, substrates for use according to the present invention include proteins, peptides, amino acids and their derivatives such as: antibodies and fragments thereof; cytokines and derivatives or fragments thereof, for example, the interleukins (IL) and especially the IL-1, IL-2, IL-3, IL-4, IL-5, IL-6, IL-7, IL-8, IL-9, IL-10, IL-11 and IL-12 subtypes thereof; colony stimulating factors, for example granulocyte-macrophage colony stimulating factor, granulocyte-colony stimulating factor (alpha and beta forms), macrophage colony stimulating factor (also known as CSF-1); haemopoietins, for example erythropoietin, haemopoietin-alpha and kit-ligand (also known as stem cell factor or Steel factor); interferons (IFNS), for example IFNalpha, IFNbeta and IFNgamma; growth factors and bifunctional growth modulators, for example epidermal growth factor, platelet derived growth factor, transforming growth factor (alpha and beta forms), amphiregulin, somatomedin-C, bone growth factor, fibroblast growth factors, insulin-like growth factors, heparin binding growth factors and tumour growth factors; differentiation factors and the like, for example macrophage differentiating factor, differentiation inducing factor (DIF) and leukaemia inhibitory factor; activating factors, for example platelet activating factor and macrophage activation factor; coagulation factors such as fibrinolytic/anticoagulant agents including heparin and proteases and their pro-factors, for example clotting factors VII, VIII, IX, X, XI and XII, antithrombin III, protein C, protein S, streptokinase, urokinase, prourokinase, tissue plasminogen activator, fibrinogen and hirudin; peptide hormones, for example insulin, growth hormone, gonadotrophins, follicle stimulating hormone, leutenising hormone, growth hormone releasing hormone and calcitonin; enzymes such as superoxide dismutase, glucocerebrosidase, asparaginase and adenosine deaminase; vaccines, for example hepatitis-B vaccine, malaria vaccine, melanoma vaccine and HIV-1 vaccine; transcription factors and transcriptional modulators; carbohydrates, glycosoaminoglycans, glycoproteins and polysaccharides; lipids, for example phosphatidylethanolamine, phosphtidylserine and derivatives thereof; sphingosine; and derivatives thereof; nucleotides, nucleosides, heterocyclic bases, DNA, RNA, synthetic and non-synthetic oligonucleotides including those with nuclease resistant backbones; vitamins; antibiotics including lantibiotics; bacteristatic and bactericidal agents; antifungal, anthelminthic and other agents effective against infective agents including unicellular pathogens; small effector molecules such as noradrenalin, alpha adrenergic receptor ligands, dopamine receptor ligands, histamine receptor ligands, GABA/benzodiazepine receptor ligands, serotonin receptor ligands, leukotrienes and triodothyronine; cytotoxic agents such as doxorubicin, methotrexate and derivatives thereof.

The substrate may also be part of a larger multi-molecular structure. These include cells or parts thereof, for instance erythrocytes, erythrocyte "ghosts" and leukocytes, viruses, unicellular organisms, liposomes such as multilamellar vesicles and unilamellar vesicles, micelles and micelle-like structures, and aggregates, microemulsions, coacervates, emulsions and suspensions of the foregoing. The substrate may also be a surface on a device such as a catheter, stent, contact lens or artificial valve.

It will be appreciated that when the substrate is part of such a structure there will generally be many reactive groups in each structure; treatment according to the invention may therefore produce a structure bearing many PEG moieties. When the PEG is bi- or multi-valent, reaction with a multimolecular substrate may result in intermolecular cross-linking by the PEG between molecules of the same target structure and/or between molecules of different target structures as well as intramolecular bonding of the PEG to more than one position on the same molecule of a target structure.

Substrates lacking a reactive group may be modified so as to create one or more reactive groups; this is within the ability of those skilled in the art and can be achieved by well-known techniques.

Some substrates (e.g. RNA and single stranded DNA) pose special problems because they may provide too many reactive groups to which the PEG would attach in a standard reaction. Therefore, if desired, some groups may be temporarily protected by involvement in an appropriate conformation precluding nucleophilic attack on the halogenated PEG, as for example by the hydrogen bonding associated with base pairing of DNA (see below).

The term "blocking group" as used herein is intended to imply a moiety which when covalently bound to a PEG terminus, is capable of preventing the attachment of an activating group to that terminus during the activation process.

An embodiment of the present process involves site-specific modification of DNA, RNA and synthetic oligonucleotide targets (or of any molecule containing and amino or other reactive group which can participate in interactions such as hydrogen bonding with another molecule or compound) by precluding nucleophilic attack on the halogenated PEG species by reactive groups on the target. The bases adenine (A), cytosine (C) and guanine (G) [but not uracil (U) or thymine (T)] provide suitable targets in DNA, RNA and synthetic oligonucleotides for modification with PEG moieties according to the invention and thus these are special targets with the problem that there may be too many available reactive groups to which the polymer can be attached. By using various restriction fragment DNA cleavage sites as a model system, selected bases A, C or G can be modified by the expedient of leaving short stretches (e.g. 2-4 bases) single stranded. Adenine bases appear to be the most susceptible to such modification. Blunt ended double stranded DNA is not readily coupled under the conditions described, indicating that hydrogen bonding between base pairs is sufficient to preclude interaction of the amino groups of A, C and G bases with the activated PEG.

Site-specific DNA modification by polymer can be achieved by the expedient of including one or more A, C or G bases in a short single stranded section of DNA by appropriate restriction enzyme digestion or by hybridising oligonucleotides of dissimilar lengths with the DNA to protect bases which are not to be modified or by exploiting the natural strand asymmetry of polymerise chain reaction products which have a one base-pair overhand, or by exploiting localised regions of single strandedness achieved by natural or artificial localised melting of the double helix. The reaction of methoxyPEGBr with 5-FU is not included within the present invention.

The term "medical therapy" as used herein includes therapeutic, diagnostic and prophylactic regimes.

Non-limiting examples of the invention will now be described with reference to the accompanying Figures, which show:-
- **FIGURE 1**: Reverse phase liquid chromatography elution profiles of a) MPEG-Cl; b) MPEG and c) a mixture of MPEG-Cl and MPEG.
- **FIGURE 2**: a) A representative elution profile of the reaction products of the PEGylation of lysozyme using MPEG-Cl, separated on a Superose 12 column.
b) A further example of a similar experiment to that shown in Figure 2a
- **FIGURE 3**: A representative elution profile of reaction products of the PEGylation of lysozyme using TMPEG, separated on a Superose 12 column.
- **FIGURE 4**: Elution profiles on Superose 12 of reaction products of PEGylation of lysozyme by MPEG-Cl at alkaline pH.
- **FIGURE 5**: Elution profiles on Superose 12 of reaction products of PEGylation of lysozyme by MPEG-Cl at a) 4°C for 21min and b) 4°C for 72.5h.
- **FIGURE 6**: Elution profiles on Superose 12 of reaction products of PEGylation of lysozyme using TMPEG at 3°C, 15.5°C and 22.5°C.
- **FIGURE 7**: Reverse phase liquid chromatography of polymer species (MPEG,MPEG-Cl and TMPEG in peaks 1-3 respectively) formed using the procedure set out in example 5.
- **FIGURE 8**: The elution profile from a Superose 12 column (using a computerised FPLC) of the reaction products of the PEGylation of lysozyme using MPEG-Cl produced by the method of example 5.
- **FIGURE 9**: The elution profile from a Superose 12 column (using a computerised FPLC) when the amount of TMPEG is equivalent to 10% of the total activated polymer used in Figure 8 above (i.e. similar to the level of contaminating TMPEG in the sample used for PEGylation in Figure 8).
- **FIGURE 10**: The elution profile from a Superose 12 column (using a computerised FPLC) of the reaction products of the PEGylation reaction of lysozyme described in Example 6; i.e. using MPEG-C1, produced by the method of Example 5, from which traces of TMPEG had been removed by prolonged hydrolysis as set out in Example 6.

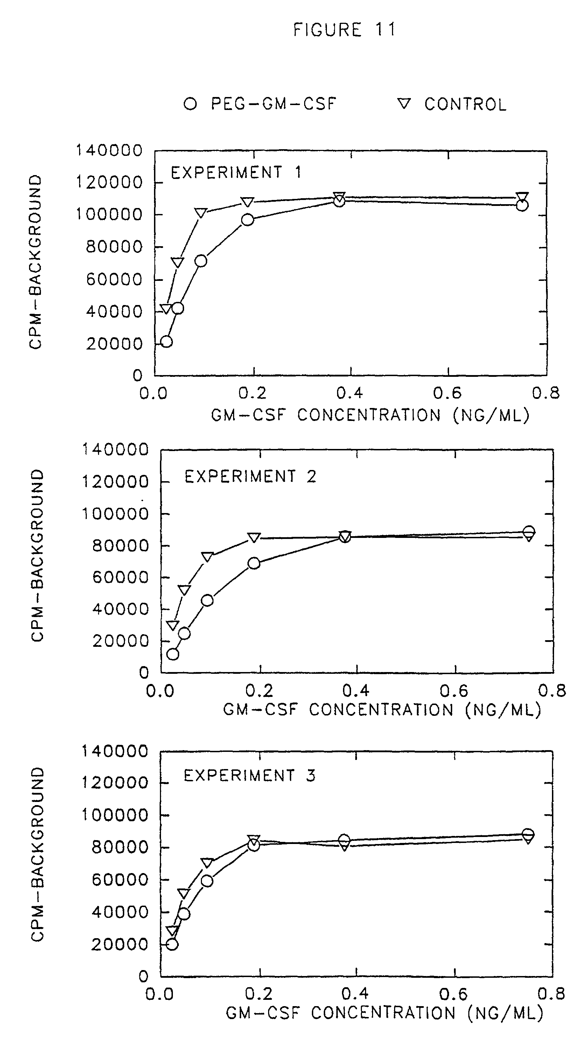

- **FIGURE 11**: Dose response curves of PEGylated GM-CSF produced using MPEG-Cl synthesised as set out in Example 5 and sham-treated controls exposed to MPEG. The results shown in panels a, b and c are from three independent experiments.
- **FIGURE 12**: Dose response curves of PEGylated EPO produced using MPEG-Cl synthesised as set out in Example 1 and a sham-treated control exposed to MPEG.
- **FIGURE 13**: Dose response curves of PEGylated EPO produced using MPEG-Cl synthesised as set out in Example 5 and sham treated controls exposed to MPEG. Panels a-c show three independent experiments.
- **FIGURE 14**: Acid elimination by MPEG-Cl at pH 7 and Fluoride measurements (for comparison with Figure 15).
- **FIGURE 15**: Fluoride and acid elimination by TMPEG at pH 7: a) TMPEG produced by the method of [WO 95/06058]; b,c) TMPEG produced by two alternate manufacturing procedures.
- **FIGURE 16**: Acid elimination for MPEG-Cl at pH 9 and Fluoride measurements for comparison with Figure 17.
- **FIGURE 17**: Fluoride and acid elimination for TMPEG at pH 9.

### EXAMPLES

### EXAMPLE 1 Preparation and characterisation of MPEG chloride

MPEG-chloride was synthesised as described by a modification of the method of *Bayer et al (1982).* Methoxypolyethylene glycol (Molecular weight 5000, Shearwater Polymers Inc) (5g) was refluxed on an oil bath with twice redistilled thionyl chloride (10ml) for 15h under nitrogen. The thionyl chloride was removed by distillation. 5ml of dry toluene (molecular sieve, 3A, BDH) was added and distilled off. 5ml of dry dichloromethane (molecular sieve, 3A, BDH) was then added and distilled off. The residue was dissolved in 20 ml of dry dichloromethane and 200ml of dry ether was added at room temperature and the mixture stirred in an ice bath. After storage overnight at -20°C, the white solid was removed by filtration, redissolved in 20 ml of dry dichloromethane and reprecipitated with 200ml of dry ether. The precipitate was removed by filtration and dried in vacuo *(Yield 3.8 g).*

The product showed a single peak on reverse phase liquid chromatography that was distinct from the single peak shown by the MPEG starting material (Figures la-c), indicating complete derivatisation of the starting MPEG to MPEG-Cl.

All the samples were analysed as 0.2% w/v solutions in 30% CH₃CN/70%H₂O on a reverse phase column PLRP-S 100A 5µ from Polymer Laboratories, using 30 to 100% CH₃CN gradient. The elution conditions were as follows: using a flow rate of 0.5ml/minute, 30-50% CH₃CN over 20 minutes, then 50-100% CH₃CN over 2 minutes, held at 100% CH₃CN for 3 minutes under isocratic conditions, reverted back to 30% CH₃CN over 1 minute, and finally held at 30% CH₃CN for 5 minutes. The sample was injected via a 20µl loading loop. An evaporative Mass Detector (PL-EMD 960; Polymer Laboratories) at 85°C with gas flow at 5.5 litres/minute, was used to monitor the samples.

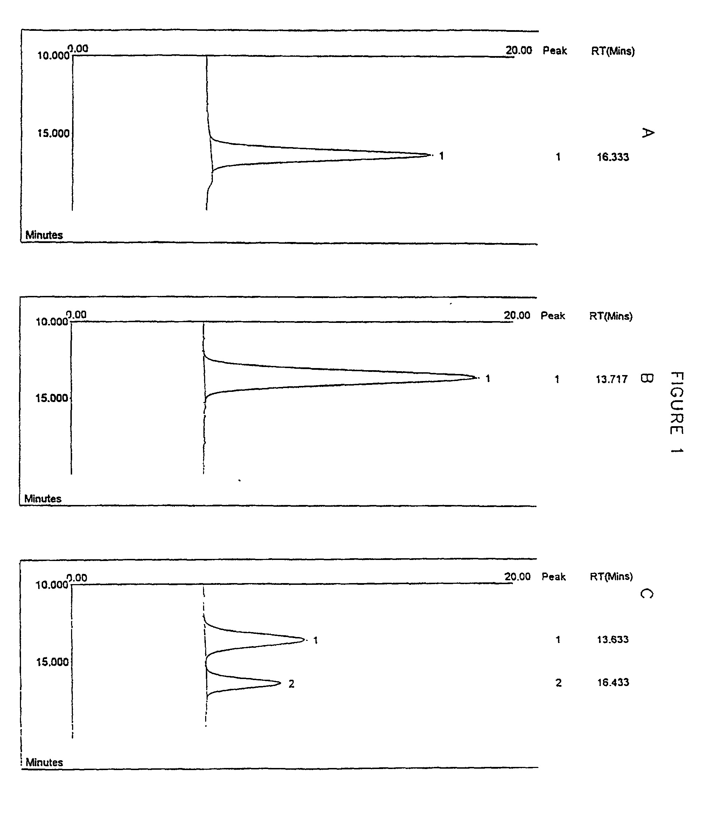

Figure 1a shows a typical elution profile of MPEG-Cl. A major peak is seen eluting with a retention time at 16.3 min (range 15.9-16.3 min in three experiments). Figure 1b shows a typical elution profile of MPEG-5K, the starting material for preparation of MPEG-Cl). A single peak is seen eluting with a retention time of circa 13.7 min (range 13.0-13.7 in five experiments). Figure 1c shows the elution profile of a mixture of the MPEG-Cl and MPEG-5K samples, by pooling equal volumes of MPEG-Cl and MPEG-5K used to produce the profiles in Figures 1a and 1b above. Two well resolved peaks are seen with retention times corresponding closely to those obtained in Figures 1a and 1b.

1H nmr of the MPEG-Cl in d₆-DMSO showed an absence of -OH signal (which is typically seen around at 4.56ppm for MPEG in DMSO). A complex multiplet centred around 3.7 ppm was consistent with literature values for O-CH2-CH2-Cl.

### Example 2 PEGylation of lysozyme

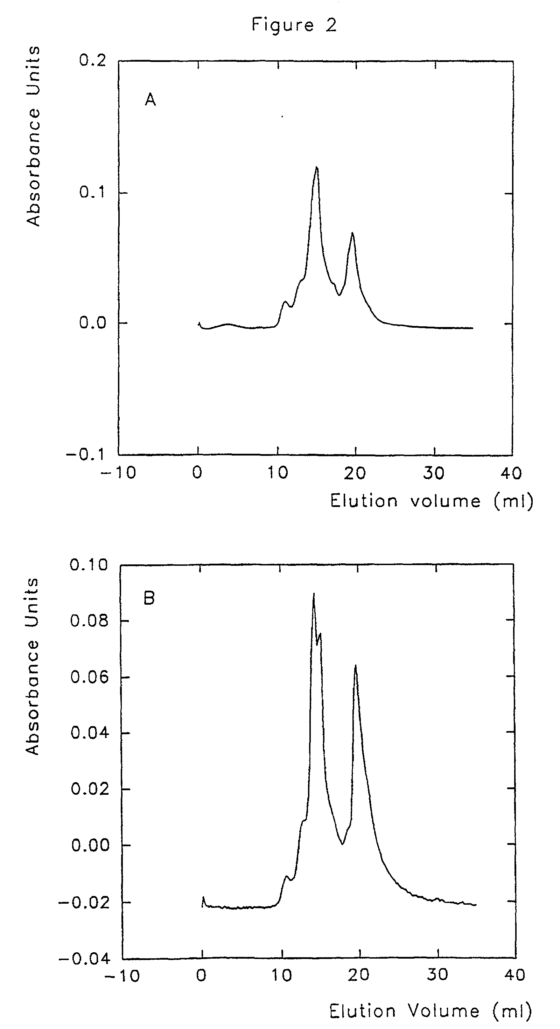

51.25mg of activated MPEG-Cl as prepared in (Example 1) was reacted with 0.466ml of 1mg/ml lysozyme (Fluka) in phosphate buffer (20mM, pH 7.0) for 21 minutes at 27°C. The initial polymer concentration was 110 mg/ml. 100ul of the reaction mixture was diluted with 400ul of PBS buffer and then 200ul was loaded onto a Superose 12 column fitted to a computerised FPLC system from Pharmacia (Sweden). The column was eluted with 50ml of PBS buffer at a flow rate of 0.3ml/min with continuous UV monitoring (214nm) at the outlet. The sensitivity of the UV detector was set at 0.5 absorbance units. The unreacted lysozyme eluted at circa 19.52ml and the PEGylated lysozyme conjugates eluted at circa 10.79, 13.02, 14.94 (Figure 2a). The chromatogram indicates that significant reaction occurred between the MPEG-Cl and lysozyme under the above conditions. This reactivity rate was reproduced in further independent experiments.

For example, 48.15mg of MPEG-Cl was reacted with 0.438ml of lmg/ml lysozyme (Fluka) in phosphate buffer (20mM, pH 7.0) for 21 minutes at 28°C. The initial polymer concentration was 109 mg/mL. 100ul of the reaction mixture was diluted with 400ul of PBS buffer and then 200ul was loaded in a Superose 12 column fitted to a computerised FPLC system from Pharmacia (Sweden). The column was eluted as for the example above except that the sensitivity of the UV detector was set at 1.0 absorbance units. The unreacted lysozyme eluted at circa 19.65ml and the PEGylated lysozyme conjugates are eluted at circa 10.49, 12.70, 14.32, 15.17 (Figure 2b). Again, the chromatogram indicates that significant reaction occurs between the MPEG-Cl and lysozyme under the above conditions.

### Comparative example 2: PEGylation of lysozyme with tresyl monomethoxy PEG as the activated polymer

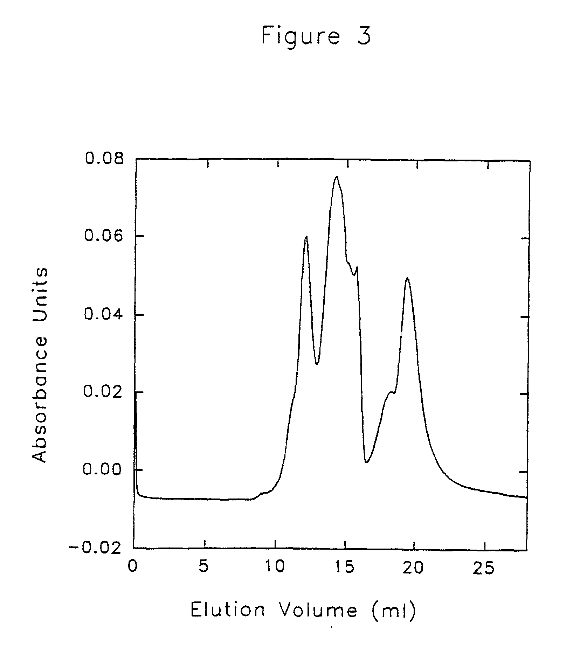

68.3 mg of tresylated MPEG (TMPEG), prepared as previously described [WO 95/06058], was reacted with 0.580 mg of lysozyme in a total volume of 0.580 ml of 20mM phosphate buffer, pH 7, for 21 minutes at 23°C. An aliquot, (100ul) of the reaction mixture was diluted with 400ul of PBS and 200ul was loaded, within a further 1 minute, onto a Superose 12 column fitted to a computerised FPLC system from Pharmacia (Sweden). The column is eluted with 25 ml of PBS at a flow rate of 0.3 ml/min with continuous UV monitoring (214 nm) at the outlet. The sensitivity of the UV detector was set at 0.2 absorbance units. The unreacted lysozyme eluted at 19.40 ml and the PEGylated lysozyme conjugates are eluted at circa 12.05, 14.24 and 15.74 ml (Figure 3). From the profile it is evident that the PEGylation reaction is occurring at a similar rate to that achieved with the MPEG-Cl sample in Example 2, which is surprising, given the anticipated low reactivity of the PEG-Cl.

### Example 3 PEGylation of lysozyme at alkaline pH.

One disadvantage of the TMPEG method overcome by the present invention is that with the former, if the activated PEG is exposed to high pH, fluoride elimination occurs. This has two consequences: first the activated polymer is rapidly exhausted and, second, a proportion of the linkages made between the polymer and the target molecule will have an alternate linkage (a sulphonate amide linkage as opposed to a secondary amine linkage). This linkage alters surface charge; introduces a coupling moiety into the product and the coupling between the polymer and target molecule or structure is unstable, particularly at alkaline pH. MPEG-Cl does not break down as rapidly as TMPEG at alkaline pH (see Example 9 below).

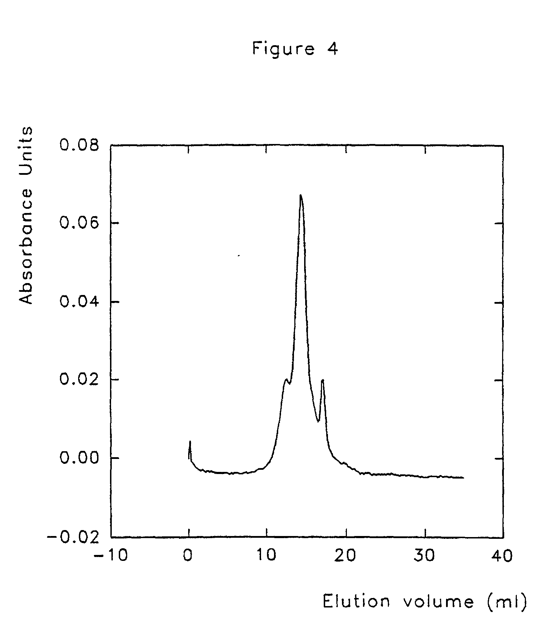

50.0 mg of activated MPEG-Cl was reacted with 0.455 ml of lmg/ml lysozyme (Fluka) in phosphate buffer (pH 8.66) for 21 minutes at 26°C. 100ul of the reaction mixture was diluted with 400ul of PBS buffer and then 200ul was loaded in a Superose 12 column and eluted as in Example 2. The chromatogram shows that one peak elutes at circa 17.03 ml and other peaks eluted at circa 12.64, 14.32 (largest) (Figure 4). There was no peak at the location of unmodified lysozyme (circa 19.2ml) indicating that the MPEG-Cl and lysozyme react significantly faster under the above conditions. It should be noted that there is no basis for the formation of an alternate linkage with the MPEG-Cl method.

### Example 4: PEGylation of lysozyme at 4°C

One further disadvantage of the TMPEG method overcome by the present invention is that, with the former, if the activated PEG is used at low temperature, longer PEGylation times and/or higher polymer concentrations are required to achieve the same degree of PEGylation as achieved at room temperature. With TMPEG, however, the duration of the PEGylation reaction cannot be much prolonged since the activated polymer hydrolyses at a significant rate (see Example 9 below). The ability to PEGylate substrates at low temperatures can be of advantage with target molecules or structures that are unstable at higher temperatures. In addition, polymer concentrations can be lowered significantly if longer reaction times are feasible.

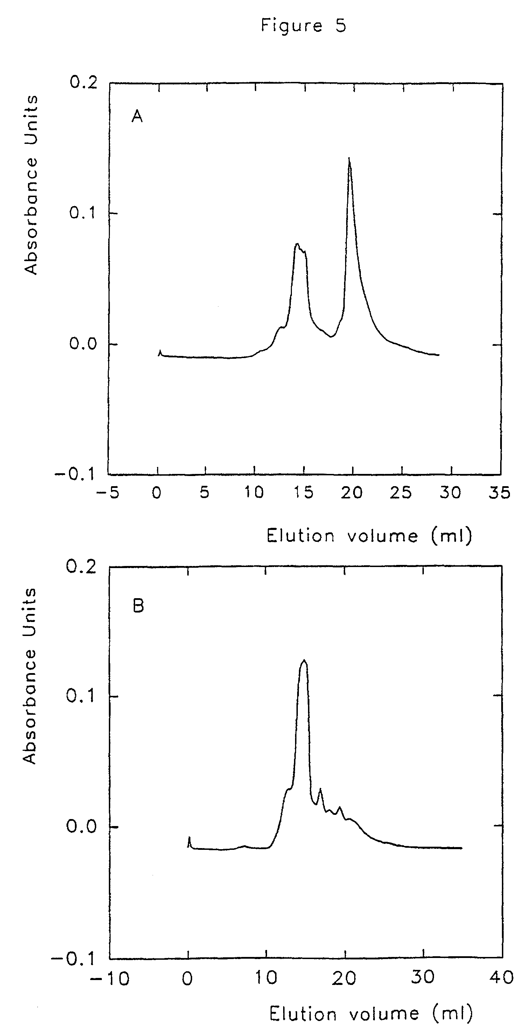

49.80mg of MPEG-Cl was reacted with 0.453mL of lmg/ml lysozyme (Fluka) in phosphate buffer (20mM, pH 7.0) for 21 minutes at 4°C. The initial polymer concentration was 110 mg/mL. 100ul of the reaction mixture was diluted with 400ul of PBS buffer and then 200ul was loaded on a Superose 12 column and eluted as in Example 2. The unreacted lysozyme eluted at circa 19.55ml and the PEGylated lysozyme conjugates eluted at circa 10.61, 12.61, 14.28, 15.10 (Figure 5a). The chromatogram indicates that although some reaction has occurred between the MPEG-Cl and the lysozyme the proportion of unmodified material is higher than was observed with similar reactions carried out at room temperature (see Figures 2a and 2b).

However, this reaction could be prolonged until essentially all lysozyme had reacted. 47.89mg of MPEG-Cl was reacted with 0.435mL of 1mg/ml lysozyme (Fluka) in phosphate buffer for 72.5 hours at 4°C. As above, The initial lysozyme concentration was 110 mg/ml. 100ul of the reaction mixture was diluted with 400ul of PBS buffer and then 200ul was loaded in a Superose 12 column and eluted as in Example 2. The unreacted lysozyme was indistinguishable among three fragments at 16.91, 18.02 and 19.31. The PEGylated lysozyme conjugates eluted at circa 12.72 and 14.86 (Figure 5b). The chromatogram indicates that almost complete reaction has occurred between MPEG-Cl and lysozyme.

### Comparative example 4: PEGylation using TMPEG at reduced temperature.

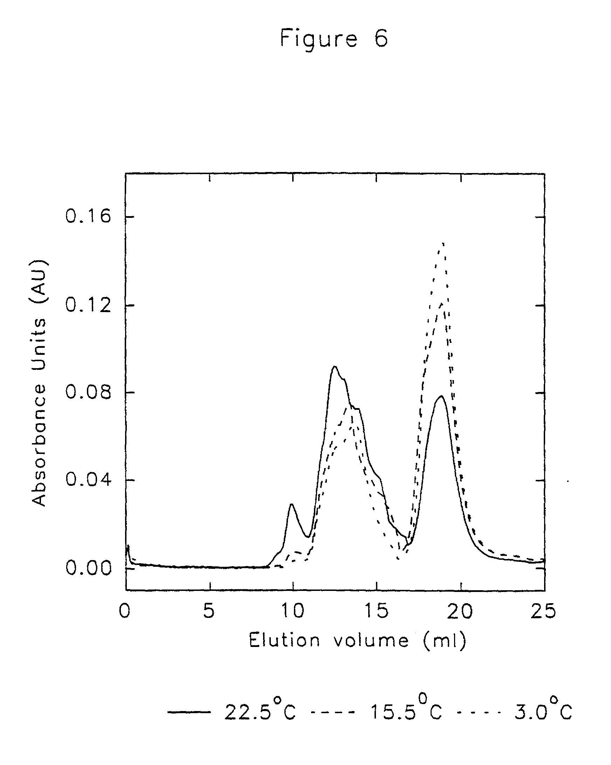

Figure 6 shows the result of reaction of lysozyme with TMPEG at three different reaction temperatures.

135mg of TMPEG-12K was reacted with 0.5ml of 1mg/ml lysozyme (Fluka) in phosphate buffer for 21 minutes. The reaction was performed three times at different temparatures: 3°C, 15.5°C and 22.5°C. 100ml of each reaction mixture was diluted with 400ul of PBS buffer and then 200ul was loaded on a Superose 12 column and eluted as in Example 2.

The estimated areas under the curve for the unmodified lysozyme peak were 52.5% at 3°C, 46.5% at 15.5°C and 23% at 22.5°C.

### Example 5: An additional synthetic route for MPEG-Cl

MPEG-Cl is also produced by variation of the previously reported manufacturing procedure for TMPEG [WO 95/06058]. This product is exposed to more rigorous washing steps than MPEG-Cl derived from the thionyl chloride method and is included here because this may be the basis of the observed superior retention of bioactivity.

MPEG (Mr 5000; 18g; Shearwater Polymers Inc, USA) was dissolved in toluene (40ml) and the water-organic azeotrope was distilled off, followed by the bulk of the toluene (109-110°C), obtaining about 35 ml of distillate. The remaining toluene was removed by rotary evaporation under reduced pressure.

The dried MPEG was dissolved in a dry acetonitrile (40ml; dried overnight with molecular sieve 3A (3Angstrom), BDH, UK, added at 10g per 50ml) at room temperature and then cooled in a water-ice bath to 1°C and magnetically stirred. One ml of ice-cold pyridine (BDH, UK) was added over 1 min with constant stirring Tresyl chloride (1ml; Fluka AG, Switzerland) was then added drop-wise to the stirred solution over 5 min. The solution was then placed at room temperature and stirring was continued for a further 2 h. Acetonitrile was then removed under reduced pressure with occasional warming with a 70°C water bath.

The solid product was dissolved in methanol-HCl (300ml: prepared using 0.75ml conc HC1 to 2.51 methanol) and cooled to -20°C overnight. The white precipitate was collected by centrifugation at 0°C and redissolved in 200ml of methanol-HCI. The solution was cooled in ice/salt for 30min and the precipitate isolated by centrifugation. To free the sample of pyridine, the process was repeated until the absorbance of the supernatant at 255nm was at a minimum. Typically 12 washes are required and the minimum absorbance (1cm path length) is 0.02, in this instance, the minimum was 0.04 and 14 washes were used without further improvement. The sample was then dissolved in methanol (200ml) and reprecipitated twice before being dried by rotary evaporation, and then overnight in a freeze dryer (yield 16g).

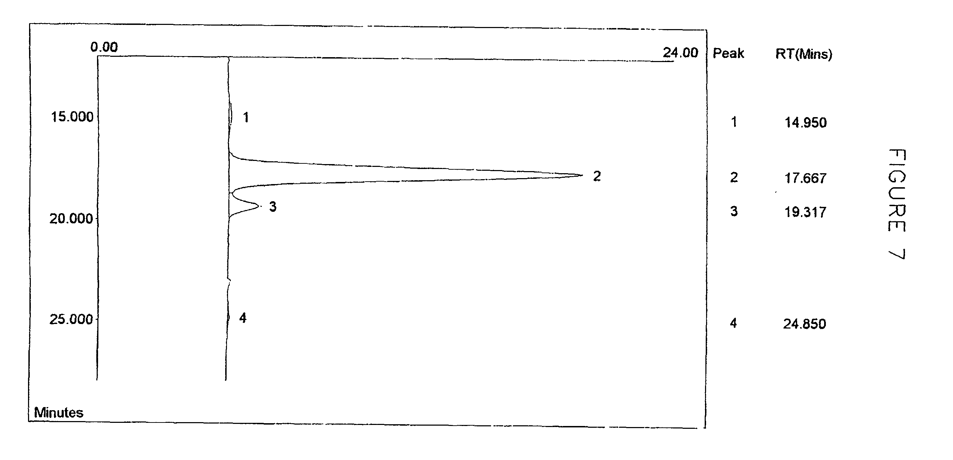

Analysis by reverse phase liquid chromatography was performed as in Example 1 using a Polymer Laboratories PLRP-6 column and a PL- EMD960 mass detector (Figure 7). The product contained negligible amounts of MPEG (<1%, see peak at 15.0min) and of the activated PEG species 92.6% was MPEG-Cl (peak at 17.7min) and 7.4% was TMPEG (peak at 19.3min) 1H-nmr and 19F-nmr showed the sample to be substantially MPEG-Cl with some TMPEG. Elemental analysis detected chlorine 0.58% (theoretical chlorine content for 100% MPEG-Cl of molecular weight 5K is 0.7%)

### Example 5A Preparation of PEG protein conjugates

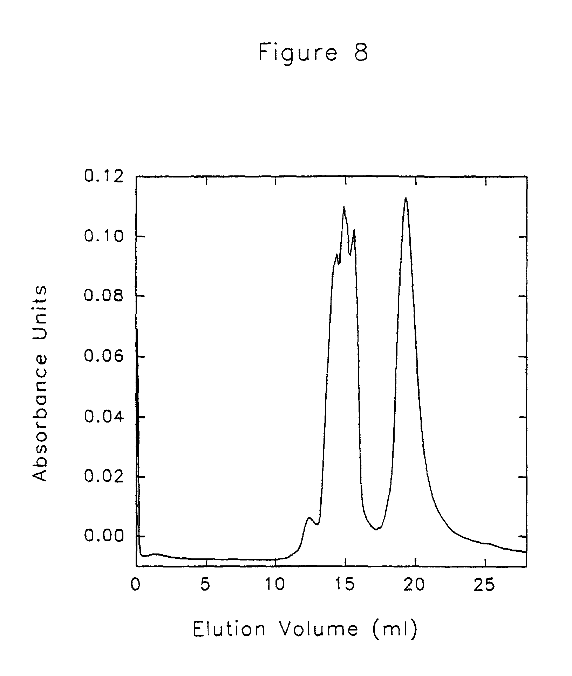

70.4 mg of MPEG-Cl produced by the method of Example 5 were reacted with 0.640 mg of lysozyme in a total volume of 0.640 ml of 20mM phosphate buffer, pH 7, for 21 minutes at 23°C. An aliquot, (100ul) of the reaction mixture was diluted with 400ul of PBS and 200ul was loaded, within a further 3 minutes, onto a Superose 12 column fitted to a computerised FPLC system from Pharmacia (Sweden). The column was eluted with 25 ml of PBS at a flow rate of 0.3 ml/min with continuous UV monitoring (214 nm) at the outlet. The sensitivity of the UV detector was set at 0.2 absorbance units. The unreacted lysozyme is eluted at circa 19.28 ml and the PEGylated lysozyme conjugates eluted at circa 12.38, 14.38, 14.88 and 15.64 ml (Figure8).

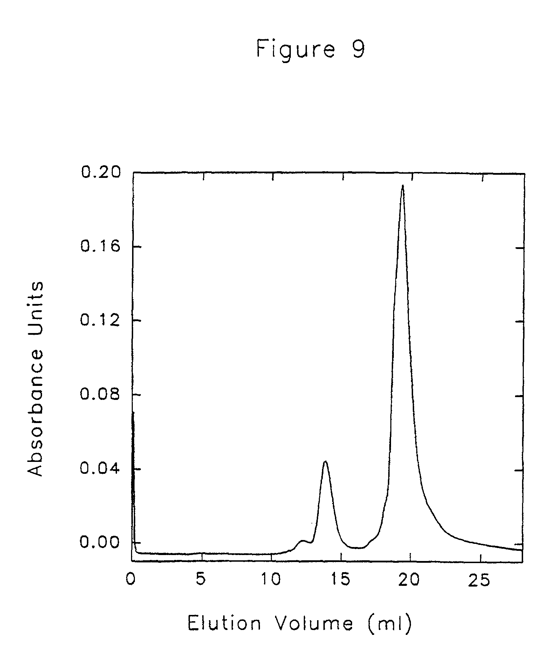

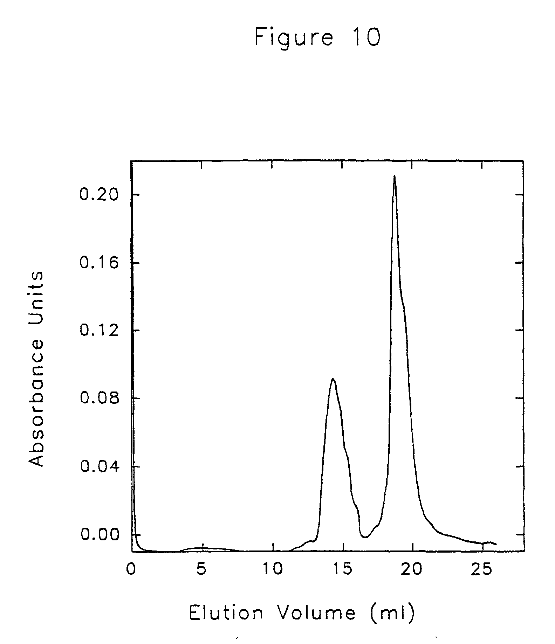

Thus reaction with lysozyme (Figure 8) showed only a slightly lower reactivity than the MPEG-Cl prepared as in Example 1 and reacted with lysozyme in Figures 2a and b. That the reactivity was due to the MPEG-Cl and not the contaminating TMPEG was demonstrated in two ways: first by showing that an equivalent amount of TMPEG to that contaminating the MPEG-Cl preparation had a much lower reactivity (comparative Example 5 Figure 9), and second, that after prolonged hydrolysis sufficient to convert the residual TMPEG to MPEG the remaining MPEG-Cl still reacted (Example 6, Figure 10).

### Comparative example 5: PEGylation of lysozyme with tresyl monomethoxy PEG as the activated polymer

6.8 mg of tresylated MPEG (92.2% purity, with 8.8% MPEG-C1 and negligible MPEG; assessed by reverse phase chromatography using a Polymer Laboratories PLRP-6 column and a PL-EMD960 mass detector) was reacted with 0.580 mg of lysozyme in a total volume of 0.580 ml of 20mM phosphate buffer, pH 7, for 21 minutes at 23°C. An aliquot, (100ul) of the reaction mixture was diluted with 400ul of PBS and 200ul was loaded, within a further 1 minute, onto a Superose 12 column fitted to a computerised FPLC system from Pharmacia (Sweden). The column is eluted with 25ml of PBS at a flow rate of 0.3 ml/min with continuous UV monitoring (214 nm) at the outlet. The sensitivity of the UV detector was set at 0.2 absorbance units. The amount of tresylated MPEG used in this reaction was reduced ten fold relative to the MPEG-Cl preparation used in example 5 (i.e. to an amount slightly more than the contaminating TMPEG present in Example 5), however much less PEGylation was observed (Figure 9) than that observed in the MPEG-C1 example (Figure 8), despite the TMPEG exposure being similar. Thus showing that the reaction in Example 5 was due to the MPEG-Cl.

### Example 6: Preparation of PEG-protein conjugates after hydrolysis of residual TMPEG in the MPEG-Cl sample of Example 5.

An aliquot of the MPEG-C1 sample (105mg) prepared in Example 5 was subjected to hydrolysis in water (525ul) for 17 days. After this the sample contained 82.4% MPEG-Cl, 0% TMPEG and 17.6% MPEG (assessed by reverse phase chromatography using a Polymer Laboratories PLRP-6 column and a PL-EMD960 mass detector).

275 ul of this sample was reacted with 0.5mg of lysozyme in a total volume of 0.5 ml of 20mM phosphate buffer, pH7 for 21 minutes at 23°C. An aliquot (100ul) of the reaction mixture was diluted with 400ul of PBS and 200ul was loaded, within a further 1-2 minutes, onto a Superose 12 column fitted to a computerised FPLC system from Pharmacia (Sweden). The column was eluted with 25ml of PBS at a flow rate of 0.3 ml/min with continuous UV monitoring (214 nm) at the outlet. The sensitivity of the UV detector was set at 0.2 absorbance units. The unreacted lysozyme eluted at 18.75ml and the PEGylated lysozyme conjugates are eluted as a shouldered peak at circa 14.33ml with two subsidiary shoulders evident at slightly higher elution volumes (Figure 10). From the profile it is evident that the PEGylation reaction is surprisingly, given the theoretically anticipated very low reactivity of MPEG-C1, still occurring at a significant rate despite the absence of any TMPEG and despite conversion of a proportion of the MPEG-Cl to MPEG.

### Example 7: PEGylation of GM-CSF and retention of bioactivity

10 ul of GM-CSF (Hoescht) at 10 ug/ml in PBS were mixed with 15 ul of a solution of MPEG-Cl (produced by the method of Example 5) at circa 250 mg/ml and 15 ul of sterile PBS (Gibco) in a sterile eppendorf tube. Sham treatment controls were set up with MPEG-5K obtained from Union Carbide. The reaction mixture was incubated using a rotary mixer for 2 h at room temperature. It has previously been established that, given the reactivity rate of the activated polymer, these reaction conditions produce a statistical mixture of PEGylated GM-CSF products with mainly 1-3 PEG chains per molecule and over 75% modification. 8 ul of reaction mixture were then added to 10 ml of RPMI-1640 medium (containing 10% of heat inactivated Foetal Calf Serum, Life Technologies) to obtain a solution of GM-CSF at 2 ng/ml. The bioactivity of the samples was tested in thymidine uptake assays using a GM-CSF responsive cell line in 96 well microtiter plates (Nunclon). The samples were diluted with fully supplemented RPMI-1640 corrected for PBS content (64 ul of sterile PBS were added to 80 ml of RPMI-1640) in order to obtain a range of concentrations of GM-CSF from 0.05 to 0.5 ng/ml. The 150 ul solution of GM-CSF in each well received 5x10⁵ TF-1 cells (starved for 24h, i.e. grown for 24h without addition of GM-CSF) and the plate was then incubated for 18h at 37°C under 5% CO₂ atmosphere. The growth stimulation is then quantified using ³H-Thymidine incorporation. [³H]-Thymidine stock (Amersham - TRK120 - batch: B395) was 100 fold diluted and 50 ul of this solution were added to each well. The plate was further incubated for 4h at 37°C under 5% CO₂ atmosphere. The cells were harvested onto a glass filter (Wallac, size 90x120mm), the filter was dried for 2h at 75°C and the dried filter was transferred to a bag (Wallac, size 90x120mm) containing 5 ml of scintillation liquid (Wallac, Betaplate Scint.). the beta emission was quantified using a beta counter (Wallac, 1450 Microbeta plus). The data were background subtracted and CPM-background was plotted against GM-CSF concentration (ng/ml). This experiment was repeated 3 times (Figure 1 la-c). The incubation of GM-CSF with this batch of MPEG-Cl resulted in a conservation of bioactivity of 52.8% +/- 5.8%.

### Example 8 : PEGylation of Erythropoietin (EPO) and retention of bioactivity.

5 ul of EPO (Cilag) at 3200 U/ml in PBS were mixed with 29 ul of an MPEG-Cl solution at circa 250 mg/ml and 61 ul of sterile PBS (Gibco) in a sterile eppendorf tube. The MPEG-Cl was produced by the method of Example 1. Control sham-treated samples were also prepared using MPEG (obtained from Shearwater Polymers Inc.). The reaction mixture was incubated using a rotary mixer for 2 h at room temperature. 705 ul of RPMI-1640 medium (containing 10% of heat inactivated Foetal Calf Serum) (Life Technologies) were then added to 95 ul of reaction mixture to obtain a solution of EPO at 20 U/ml. The bioactivity of the samples was tested in a 96 wells microtiter plate (Nunclon). The samples were diluted with fully supplemented RPMI-1640 corrected for PBS (9.5 ml of sterile PBS were added to 70.5 ml of RPMI-1640) in order to obtain a range of EPO concentrations from 1 to 10 U/ml. The 150 ul solution of EPO in each well received 5x10⁵ TF-1 cells (starved for 24h, i.e. grown for 24h without addition of GM-CSF, the usual cell growth support for this cell line) and the plate was incubated for 18h at 37°C under 5% CO₂ atmosphere. The growth stimulation was then quantified using [³H]-Thymidine incorporation. [³H]-Thymidine stock (Amersham - TRK120 - batch: B395) was 100 fold diluted and 50 ul of this solution were added to each well. The plate was further incubated for 4h at 37°C under 5% CO₂ atmosphere. The cells were harvested onto a glass filter (Wallac, size 90x120mm), the filter was dried for 2h at 75°C and the dried filter was transferred to a bag (Wallac, size 90x120mm) containing 5 ml of scintillation liquid (Wallac, Betaplate Scint.). The beta emission was quantified using a beta counter (Wallac, 1450 Microbeta plus).

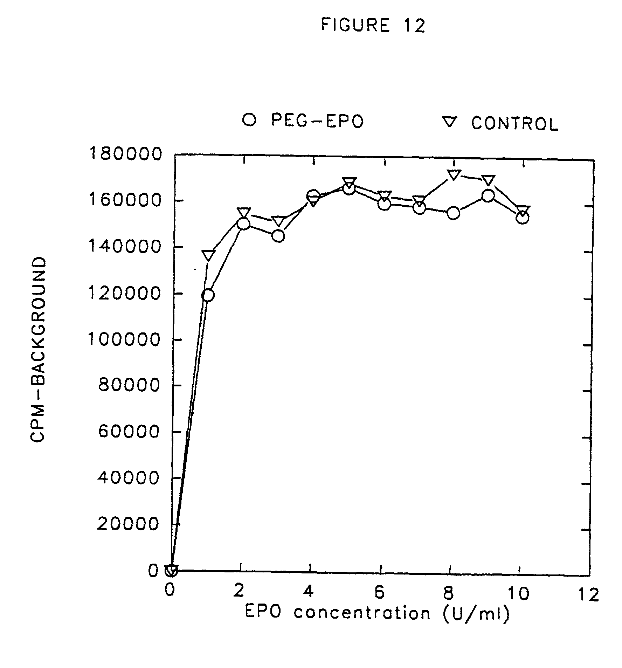

The data were background subtracted and CPM-background were plotted against EPO concentration (as U/ml, i.e. without adjusting for loss of native activity; Figure 12). There was no significant loss of bioactivity.

5 ul of EPO (Cilag) at 3200 U/ml in PBS were mixed with 29 ul of an MPEG-Cl solution at circa 250 mg/ml and 61 ul of sterile PBS (Gibco) in a sterile eppendorf tube. The MPEG-Cl was prepared as in Example 5. Sham treated control EPO was also prepared using MPEG-5K obtained from Union Carbide. The reaction mixture incubated using a rotary mixer for 2 h at room temperature. 705 ul of RPMI-1640 medium (containing 10% of heat inactivated Foetal Calf Serum, Life Technologies) were then added to 95 ul of reaction mixture to obtain a solution of EPO at 20 U/ml. The bioactivity of the samples was tested as described for Figure 12.

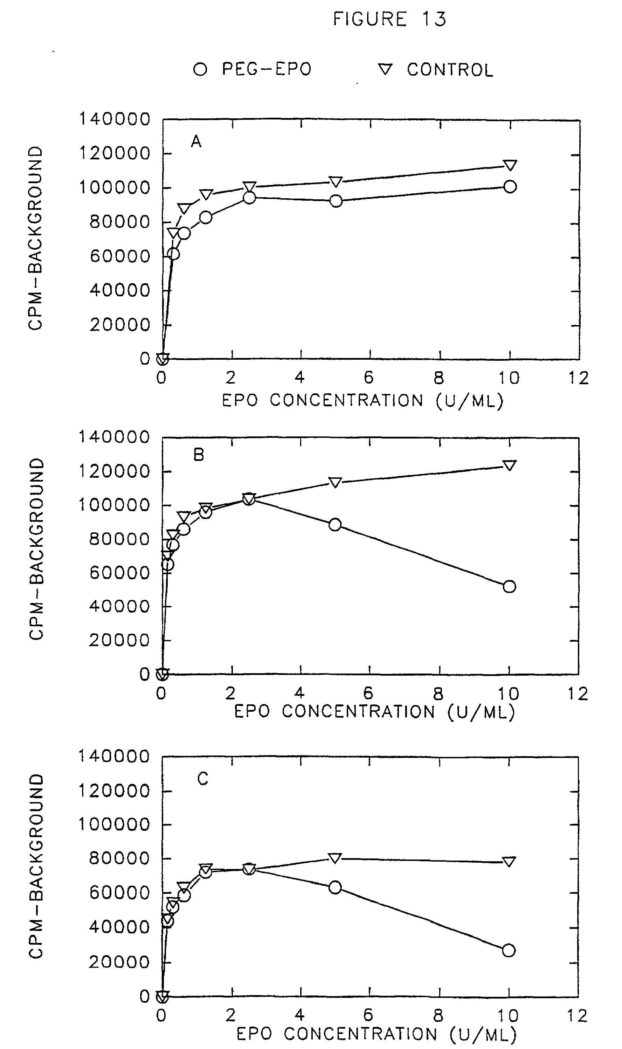

This experiment was repeated 3 times. The data were background subtracted and CPM-Background were plotted against EPO concentration (U/ml) (Figure 13a-c). At low doses of the reaction products, the dose response curves were superimposable, but at higher doses in two experiments there was progressive departure between the test and control curves. This indicates the presence of some toxic or inhibitory material (the EPO assay is particularly sensitive to inhibition, much more so than the GM-CSF assay). The level of toxicity observed is lower than for several other PEGylation procedures previously examined (cf. the cyanuric chloride method and the phenylchloroformate method *(Francis, G.E. et al (1996) J. Drug Targeting 3 321-340).* The superimposition of the upward part of the test and control dose response curves at low doses of test material indicates no significant loss of bioactivity.

### Example 9 and comparative Example 9: Breakdown of MPEG-CI and TMPEG in aqueous solution.

One advantage of the present invention is the relative stability of the activated polymer.

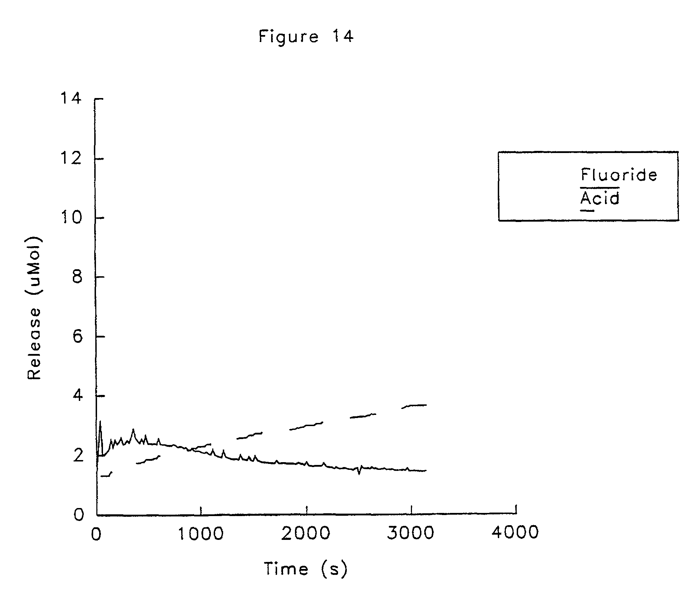

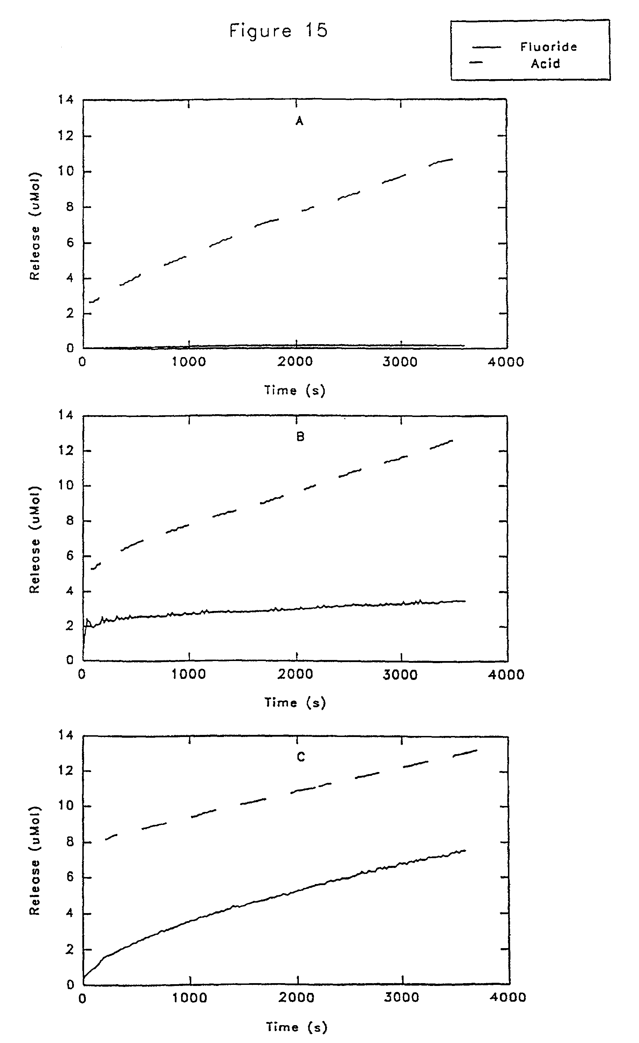

Figure 14 shows the effect for MPEG-Cl and Figure 15 a-c shows the breakdown of three TMPEG samples.

Figures 14 and 15a-c compare the breakdown rates at pH 7 for MPEG-Cl made by the method of example 1 and three TMPEG samples made by different manufacturing techniques.
The release of acid and of fluoride from samples of activated MPEGs at pH 7 and also at pH 9 were measured in a pH-stat (Mettler Toledo DL 77 titrator) fitted with a fluoride electrode (Mettler Toledo DX 219) and pH electrode (Metler Toledo DG101-SC). 25 ml of 0.9% NaCl was adjusted to pH 7 or 9 with approximately 0.01 M NaOH (standardised by potassium hydrogen phthalate titration). MPEG-Cl or TMPEG (approx 100mg; approx 20 umoles) was added to the saline and simultaneous measurements of fluoride concentration and alkali consumed were made at 20 second intervals for up to 60 min. Results are plotted as umoles of fluoride present and acid produced as a function of time. Samples of activated polymers dissolved in the NaCl required NaOH to bring to the starting pH, indicating that they were not neutral but were somewhat acidic. This immediate uptake of alkali was then followed by a steady uptake as acid was released progressively, and is seen as an intercept at zero time in the plot of alkali uptake against time. This value can be subtracted to provide a plot of acid release at the selected pH.

The changes in fluoride in Figure 14 are an artefact of the impact of OH ions on the fluoride electrode.

Figure 15a shows a sample of TMPEG prepared as previously described [WO 95/06058] with very low fluoride elimination. Note that its acid elimination is substantially higher than that of the MPEG-Cl sample. Figures 15b and c show two samples of TMPEG (obtained from Sigma and Shearwater Polymers Inc) made by different manufacturing procedures and exhibiting higher fluoride elimination. A combination of hydrolysis to MPEG and trifluoroethanesulphonic acid and fluoride elimination thus produces substantially faster breakdown of TMPEG versus MPEG-Cl. Note that MPEG-Cl left in water for 17 days still retained good reactivity (see above), implying that the acid elimination rates at acidic pH is even lower than that at pH 7.

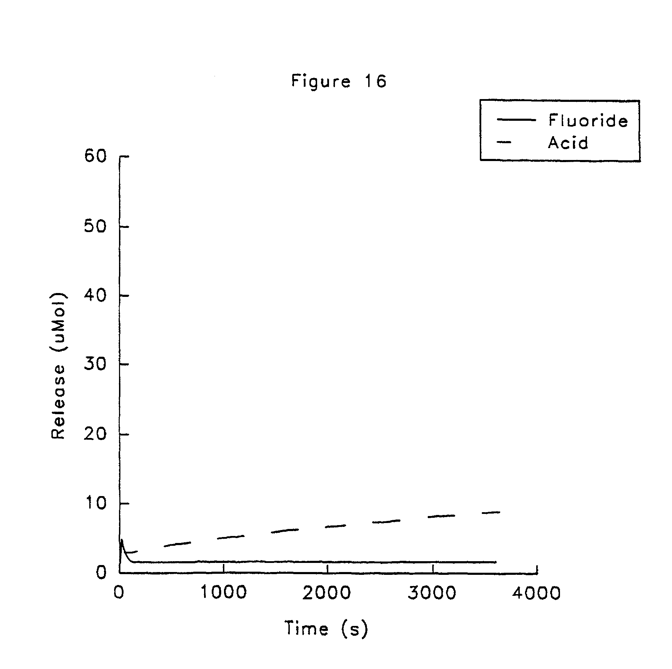

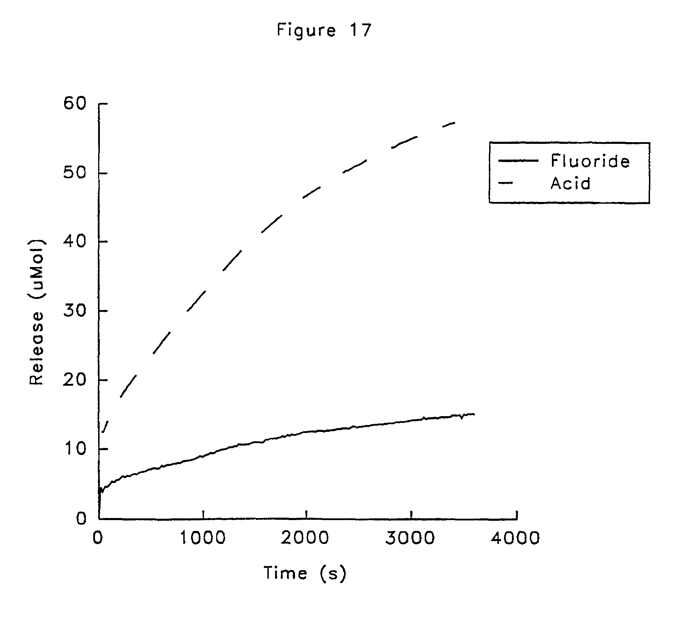

Figures 16 and 17 show comparable break down rates for MPEG-Cl and TMPEG at pH 9.0.

## Claims

1. A process for the PEGylation of a substrate, comprising the reaction of a halogenated PEG of general formula I or II
X-PEG-Y (I)
(PEG)ₙ-Yᵢ (II)
wherein:
PEG is a bivalent group of formula ―(-CH₂CH₂O-)ₘ-CH₂CH₂―, where m is ≥1, derived a polyethylene glycol; X is a halogen atom, a blocking group or an activating group capable of coupling the PEG moiety to another moiety; Y is a halogen; n is the number of PEG termini, n is ≥ 2; i is ≤n and i/n PEG termini are substituted by Y in compounds of Formula II;
with the substrate and the PEG binds directly to the substrate, with the proviso that the substrate is not a steroid or when the halogenated PEG is PEG-bromide, the substrate is not 5-fluorouracil.

2. A process according to claim 1 wherein Y is Cl, Br or I.

3. A process according to either of claims 1 or 2 wherein X is a blocking group and is methyl, t-butyl or benzyl ether.

4. A process according to either of claims 1 or 2, wherein X is a tresyl activating group.

5. A process according to either of claims 1 and 2, wherein the halogenated PEG is one of; methoxy-PEG-Cl, methoxy-PEG-Br, methoxy-PEG-I, Cl-PEG-Cl, Br-PEG-Br or I-PEG-I.

6. A process according to either of claims 1 and 2 wherein the substrate is selected from proteins, peptides, DNA, RNA, nucleotides, nucleotide analogues, hormones, antibiotics, liposomes, viruses, unicellular organisms, micelles, metallic plastic, or polymeric surfaces.

## Patentansprüche

1. Verfahren zur PEGylierung eines Substrates unter Umsetzung eines halogenierten PEG der allgemeinen Formel I oder II
X-PEG-Y (I)
(PEG)ₙ-Yᵢ (II)
worin
PEG eine zweibindige Gruppe der Formel -(CH₂CH₂O-)ₘ-, worin m > 1 ist und die von einem Polyethylenglykol sich herleitet, X ein Halogenatom, eine blockierende Gruppe oder eine aktivierende Gruppe ist, die in der Lage ist, den PEG-Rest an einen anderen Rest zu binden, Y ein Halogen ist, n die Zahl der PEG-Endgruppen ist, n ≥ 2 ist, i ≤ n ist und i/n PEG-Endgruppen durch Y in Verbindungen der Formel II substituiert sind,mit dem Substrat und unter direkter Bindung des PEG an das Substrat unter der Voraussetzung, daß das Substrat kein Steroid ist oder, wenn das halogenierte PEG PEG-Bromid ist, das Substrat nicht 5-Fluoruracil ist.

2. Verfahren nach Anspruch 1, bei dem Y Cl, Br oder I ist.

3. Verfahren nach einem der Ansprüche 1 oder 2, worin X eine blockierende Gruppe ist und Methyl, tert-Butyl oder Benzylether bedeutet.

4. Verfahren nach einem der Ansprüche 1 oder 2, bei dem X eine Tresylaktivierungsgruppe ist.

5. Verfahren nach einem der Ansprüche 1 und 2, bei dem das halogenierte PEG eines von Methoxy-PEG-C1, Methoxy-PEG-Br, Methoxy-PEG-I, Cl-PEG-Cl, Br-PEG-Br oder I-PEG-I ist.

6. Verfahren nach einem der Ansprüche 1 und 2, bei dem das Substrat unter Proteinen, Peptiden, DNA, RNA, Nucleotiden, Nucleotidanalogen, Hormonen, Antibiotika, Liposomen, Viren, einzelligen Organismen, Micellen, Metall-, Kunststoff- oder Polymeroberflächen ausgewählt wird.

## Revendications

1. Procédé pour la PEGylation d'un substrat, comprenant la réaction d'un PEG halogéné de formule générale I ou II
X-PEG-Y (I)
(PEG)ₙ-Yᵢ (II)
dans laquelle :
le terme PEG représente un groupe bivalent de formule ---(-CH₂CH₂O-)ₘ---CH₂CH₂---, dans laquelle m est ≥1, dérivé d'un polyéthylène-glycol ; X représente un atome d'halogène, un groupe de blocage ou un groupe activateur apte au couplage du groupement PEG à un autre groupement ; Y représente un halogène ; n représente le nombre d'extrémités PEG, n étant ≥2 ; i est ≤n et i/n extrémités PEG sont substituées par Y dans les composés de formule II ;
avec le substrat, le PEG se liant directement au substrat, sous réserve que le substrat ne soit pas un stéroïde ou, lorsque le PEG halogéné consiste en PEG-bromure, le substrat ne consiste pas en 5-fluoro-uracile.

2. Procédé suivant la revendication 1, dans lequel Y représente Cl, Br ou I.

3. Procédé suivant l'une des revendications 1 et 2, dans lequel X représente un groupe de blocage et consiste en éther méthylique, tertio-butylique ou benzylique.

4. Procédé suivant l'une des revendications 1 et 2, dans lequel X représente un groupe activateur trésyle.

5. Procédé suivant l'une des revendications 1 et 2, dans lequel le PEG halogéné est l'un des suivants : méthoxy-PEG-C1, méthoxy-PEG-Br, méthoxy-PEG-I, Cl-PEG-Cl, Br-PEG-Br et I-PEG-I.

6. Procédé suivant l'une des revendications 1 et 2, dans lequel le substrat est choisi entre des protéines, des peptides, l'ADN, l'ARN, des nucléotides, des analogues de nucléotides, des hormones, des antibiotiques, des liposomes, des virus, des organismes unicellulaires, des micelles, des surfaces métalliques, des surfaces de matière plastique ou des surfaces polymères.
